# EUROPEAN PATENT APPLICATION

(11) **EP 1 741 756 A1**
(43) Date of publication of application: **10.01.2007**
(21) Application number: 04772387.9
(22) Date of filing: 23.08.2004
(51) Int. Cl.: C09B 47/00, C07D 487/22

(54) **TETRAAZAPORPHYRIN COMPOUND**

(30) Priority: 27.08.2003 JP 2003302336
(71) Applicant: Yamamoto Chemicals, Inc., Yao-shi, Osaka 581-0034 (JP)
(72) Inventor: SAWANO, Bunji, c/o Yamamoto Chemicals, Inc., Yao-shi, Osaka 581-0034 (JP); MATSUMOTO, Mansuke, c/o Yamamoto Chemicals, Inc., Yao-shi, Osaka 581-0034 (JP)
(74) Representative: Perry, Robert Edward
(86) International application number: PCT/JP2004/012431
(87) International publication number: WO 2005/021658

(57) **Abstract**

A novel tetraazaporphine compound is provided which has a brilliant blue color and highly soluble in solvents.

A tetraazaporphyrin compound represented by the following general formula (1): 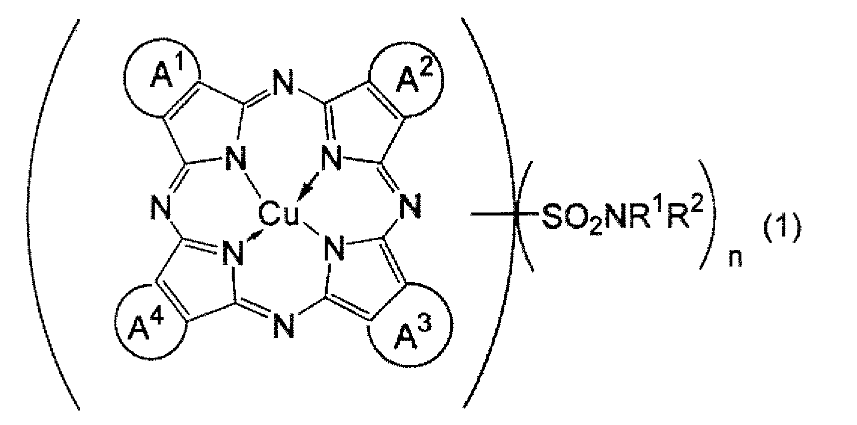 (In the formula (1), the rings represented by A¹, A², A³ and A⁴ each independently represents 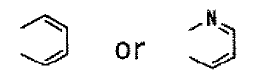 and at least one of A¹, A², A³ and A⁴ is 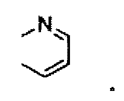 R¹ and R² each independently represents a hydrogen atom or a substituted or unsubstituted alkyl group, and n represents an integer of 1-4, provided that when one of R¹ and R² is a hydrogen atom, the other is other than a hydrogen atom. The rings A¹, A², A³ and A⁴ each independently may have a substituent(s) other than -SO₂NR¹R².)

## Description

### TECHNICAL FIELD

The present invention relates to a novel tetraazaporphyrin compounds which can be used in toners for electrophotography and inks for ink-jet printers, are excellent in solubility and have a brilliant blue color.

### BACKGROUND ART

In recent years, coloring matters or pigments excellent in solubility and having a brilliant blue color have been demanded for use in toners for full-color electrophotography and in inks for ink-jet recording.

Metallophthalocyanine compounds are used as blue pigments in various fields of application. In Japanese Kokai Publication H03-195783, for instance, use is made of metallophthalocyanine-sulfonamide compounds in inks for ink-jet recording. However, the maximum absorption wavelength of metallophthalocyaninesulfonamide compounds is around 670 nm, so that any brilliant blue color cannot be obtained.

Further, Japanese Kokai Publication H02-276866 discloses, as pigments for photorecording media, tetraazaporphine compounds although they are different from the compounds of the present invention with respect to the center atom. However, when these compounds are used in inks for ink-jet printers, for instance, the maximum absorption wavelength is found at about 650 nm and the color cannot be said to be a brilliant blue color.

Therefore, the demand for organic solvent-soluble coloring matters showing strong absorption at about 600 nm, in particular, is growing.

Recently, colorants highly soluble in polar organic solvents superior from the working environment and safety viewpoint, for example methyl lactate and ethyl lactate, have been demanded as colorants for polymers, among others.

### DISCLOSURE OF INVENTION

It is an object of the present invention to provide organic solvent-soluble tetraazaporphine compounds having a brilliant blue color and showing strong absorption at about 600 nm and, more specifically, to provide tetraazaporphine compounds which are highly soluble in organic solvents and resins and can be used in highly transparent toners and in brilliantly blue inks for ink-jet printers, among others. Another object is to provide pigments which can be used as colorants for polymer materials and are highly soluble in polar organic solvents such as methyl lactate and ethyl lactate and have a brilliant blue color.

The present inventors made intensive investigations to accomplish the above objects. As a result, they found that tetraazaporphine compounds having a specific structure show strong absorption at about 600 nm and have a brilliant blue color and are highly soluble in organic solvents and resins. Base on such findings, they have now accomplished the above objects.

Thus, the present invention provides tetraazaporphyrin compounds represented by the following general formula (1): 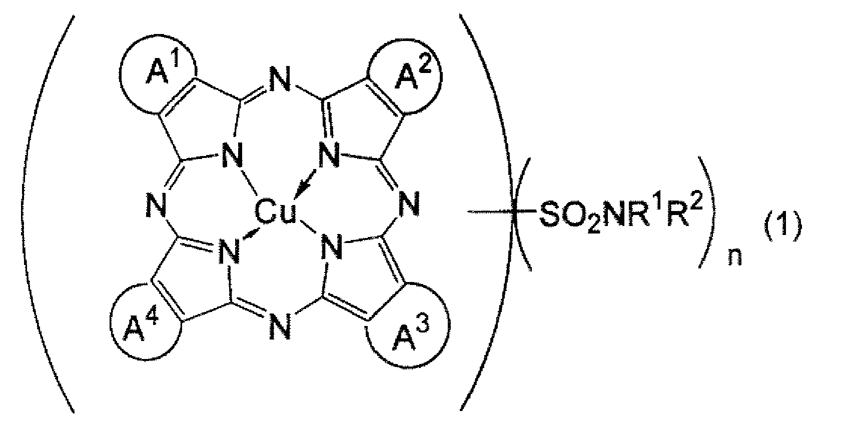 (In the above formula (1), the rings represented by A¹, A², A³ and A⁴ each independently represents 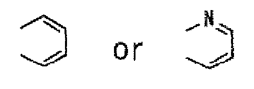 and at least one of A¹, A², A³ and A⁴ is

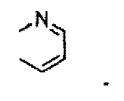

R¹ and R² each independently represents a hydrogen atom or a substituted or unsubstituted alkyl group, and n represents an integer of 1-4, provided that when one of R¹ and R² is a hydrogen atom, the other is other than a hydrogen atom. The rings A¹, A², A³ and A⁴ each independently may have a substituent(s) other than -SO₂NR¹R².)

### BRIEF DESCRIPTION OF THE DRAWINGS

[Fig. 1] This figure is a transmission spectrum of the blue crystals obtained in Example 1.

[Fig. 2] This figure is a transmission spectrum of the blue crystals obtained in Example 2.

[Fig. 3] This figure is a transmission spectrum of the blue crystals obtained in Example 3.

[Fig. 4] This figure is a transmission spectrum of the blue crystals obtained in Comparative Example 1.

### DETAILED DESCRIPTION OF THE INVENTION

In the tetraazaporphyrin compounds of the invention as represented by the above general formula (1), the unsubstituted alkyl group represented by R¹ and/or R² is preferably an alkyl group containing 1-12 carbon atoms, including such straight or branched alkyl groups as methyl, ethyl, n-propyl, isopropyl, n-butyl, isobutyl, n-hexyl, 2-ethylhexyl, n-octyl and n-dodecyl. Among them, straight or branched alkyl groups containing 4-12 carbon atoms are preferred.

As for the substituted alkyl group represented by R¹ and/or R², alkyl groups containing one or more oxygen atoms in the form of one or more ether bonds, carbonyl bonds and/or ester bonds are preferred. In particular, alkyl groups containing 2-12 carbon atoms and 1-4 oxygen atoms are preferred. As examples, there may be mentioned methoxymethyl, ethoxymethyl, butoxymethyl, methoxyethyl, ethoxyethyl, 3-methoxypropyl, 3-ethoxypropyl, 3-butoxypropyl, methoxyethoxyethyl, ethoxyethoxyethyl, butoxyethoxyethyl, methoxyethoxyethoxyethyl, ethoxyethoxyethoxyethyl, butoxyethoxyethoxyethyl, acetylmethyl, acetylethyl, propionylmethyl, propionylethyl, tetrahydrofurufuryloxymethyl, 2,2-dimethyl-1,3-dioxolane-4-methoxymethyl, 2-(1,3-dioxolane)ethoxymethyl, 2-(1,3-dioxane)ethoxymethyl, methoxycarbonylmethyl, ethoxycarbonylethyl, propoxycarbonylethyl, butoxycarbonylethyl, pentoxycarbonylbutyl, 1-(butoxymethyl)ethyl, 1-(methoxymethyl)propyl, 1-(etboxymethyl)propyl, 1-(butoxymethyl)propyl, 1-(2-methoxy-ethoxy-methyl)propyl, 1-(2-ethoxy-ethoxy-methyl)propyl, 1-(2-methoxy-2-ethoxy-2-ethoxymethyl)ethyl, 1-(2-ethoxy-2-ethoxy-2-ethoxymethyl)ethyl, 1-(2-butoxy-2-ethoxy-2-ethoxymethyl)ethyl, 1-(2-methoxy-2-ethoxy-2-ethoxymethyl)propyl, 1-(2-ethoxy-2-ethoxy-2-ethoxymethyl)propyl, 1-(2-propoxy-2-ethoxy-2-ethoxymethyl)propyl, 1-(2-butoxy-2-ethoxy-2-ethoxy-methyl)propyl, 1-(2-methoxy-2-ethoxy-2-ethoxymethyl)butyl, 1-(2-ethoxy-2-ethoxy-2-ethoxymethyl)butyl, 1-(2-propoxy-2-ethoxy-2-ethoxymethyl)butyl, 1-(2-methoxy-2-ethoxy-2-ethoxymethyl)pentyl, 1-(2-ethoxy-2-ethoxy-2-ethoxymethyl)pentyl, 1-(2-methoxy-2-ethoxy-2-ethoxy-2-ethoxymethyl)ethyl, 1-(2-ethoxy-ethoxy-2-ethoxy-2-ethoxymethyl)ethyl, 1-(2-atethoxy-2-ethoxy-2-ethoxy-2-ethoxymethyl)propyl, 1-(2-ethoxy-2-ethoxy-2-ethoxy-2-ethoxymethyl)propyl, 1-(2-methoxy-2-ethoxy-2-ethoxy-2-ethoxymethyl)butyl, 1-(2-methoxy-2-ethoxy-2-ethoxy-2-ethoxyethyl)ethyl, 1-(2-ethoxy-2-ethoxy-2-ethoxy-2-ethoxyethyl)ethyl, 1-(2-methoxy-2-ethoxy-2-ethoxy-2-ethoxyethyl)propyl, 1,1-di(methoxymethyl)methyl, 1,1-di(ethoxymethyl)methyl, 1,1-di(propoxymethyl)methyl, 1,1-di(butoxymethyl)methyl, 1,1-di(2-methoxy-ethoxymethyl)methyl, 1,1-di(2-ethoxy-ethoxymethyl), 1,1-di(2-propoxyethoxymethyl)methyl and 1,1-di(2-butoxy-ethoxymethyl)methyl.

Particularly preferred among these are those in which at least one of R¹ and R² is a group of the following formula (2): 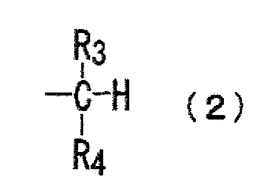 (In the formula (2), R³ and R⁴ each independently is a hydrogen atom; an unsubstituted alkyl group; an alkyl group containing one or more oxygen atoms in the form of one or more ether bonds, carbonyl bonds and/or ester bonds; an alkylcarbonyl group; or an alkoxycarboyl group, provided that at least one of R³ and R⁴ is an alkyl group containing one or more oxygen atoms in the form of one or more ether bonds, carbonyl bonds and/or ester bonds; an alkylcarbonyl group; or an alkoxycarboyl group.)

The unsubstituted alkyl group represented by R³ and/or R⁴ in the formula (2) is preferably an alkyl group containing 1-8 carbons atoms, such as methyl, ethyl, propyl, isopropyl, butyl, isobutyl, pentyl, hexyl or octyl.

As for the alkyl group containing one or more oxygen atoms in the form of one or more ether bonds, carbonyl bonds and/or ester bonds as represented by R³ and/or R⁴, alkyl groups containing 2-10 carbon atoms and 1-4 oxygen atoms are preferred. As examples, there may be mentioned methoxymethyl, ethoxymethyl, propoxymethyl, butoxymethyl, methoxyethoxymethyl, ethoxyethoxymethyl, propoxyethoxymethyl, butoxyethoxymethyl, methoxyethoxyethoxymethyl, ethoxyethoxyethoxymethyl, propoxyethoxyethoxymethyl, butoxyethoxyethoxymethyl, methoxyethoxyethoxyethoxymethyl, ethoxyethoxyethoxyethoxymethyl, propoxyethoxyethoxyethoxymethyl, butoxyethoxyethoxyethoxymethyl, acetylmethyl, propionylmethyl, tetrahydrofurufuryloxymethyl, 2,2-dimethyl-1,3-dioxolane-4-methoxymethyl, 2-(1,3-dioxolane)ethoxymethyl, 2-(1,3-dioxane)ethoxymethyl, methoxycarbonylmethyl, ethoxycarbonylmethyl, propoxycarbonylmethyl, butoxycarbonylmethyl and pentoxycarbonylmethyl.

The alkylcarbonyl group or alkoxycabonyl group represented by R³ and/or R⁴ preferably contains 2-10 carbon atoms. As examples, there may be mentioned acetyl, propionyl, propylcarbonyl, methoxycarbonyl, ethoxycarbonyl, propoxycarbonyl, butoxycarbonyl and pentoxycarbonyl.

Preferred as the tetraazaporphyrin compounds of the invention as represented by the general formula (1) are those in which R¹ and R² each independently is a hydrogen atom (provided that one of R¹ and R² is a hydrogen atom, the other is other than a hydrogen atom); an unsubstituted alkyl group; a substituted alkyl group containing one or more oxygen atoms in the form of one or more ether bonds, carbonyl bonds and/or ester bonds.

Particularly preferred are those in which R¹ and R² each independently is a hydrogen atom (provided that one of R¹ and R² is a hydrogen atom, the other is other than a hydrogen atom); an unsubstituted alkyl group containing 1-12 carbon atoms; a substituted alkyl group containing 2-12 carbon atoms and containing one or more oxygen atoms in the form of one or more ether bonds, carbonyl bonds and/or ester bonds. Among them, those in which at least one of R¹ and R² is a substituted alkyl group containing 2-12 carbon atoms and containing 1-4 oxygen atoms in the form of one or more ether bonds, carbonyl bonds and/or ester bonds are further preferred because of their high levels of solubility in polar organic solvents.

In particular, those tetraazaporghyrin compounds in which at least one of R¹ and R² is a substituted alkyl group represented by the following general formula (2): 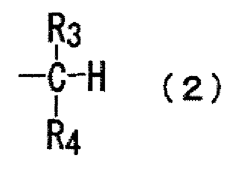 (In the formula (2), R³ and R⁴ each independently is a hydrogen atom; an unsubstituted alkyl group; an alkyl group containing one or more oxygen atoms in the form of one or more ether bonds, carbonyl bonds and/or ester bonds; an alkylcarbonyl group; or an alkoxycarboyl group, provided that at least one of R³ and R⁴ is an alkyl group containing one or more oxygen atoms in the form of one or more ether bonds, carbonyl bonds and/or ester bonds; an alkylcarbonyl group; or an alkoxycarboyl group.)

The tetraazaporphyrin compounds of general formula (1) may further have, in addition to -SO₂NR¹R², one or more substituents each selected from among alkyl groups such as methyl, ethyl and propyl, alkoxy groups such as methoxy, ethoxy and propoxy, halogen atoms such as chlorine, bromine and fluorine atoms, and a nitro group on one or more of the rings A¹ to A⁴.

The tetraazaporphyrin compounds of the invention are represented by the general formula (1) and the rings represented by A¹, A², A³ and A⁴ each is an aromatic ring represented by 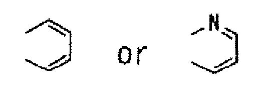 and there are a number of isomers according to the direction of ring condensation and the substitution position(s) of the substituent(s) bound thereto. More specifically, there are 5 basic ring skeleton structures represented by the formulas (3) to (7) given below, and each of them includes further isomers differing in the positions of N atoms due to the difference in the direction of pyridine ring condensation and, further, there are isomers due to different positions of substitution by a substituent(s) for the respective skeleton structures.

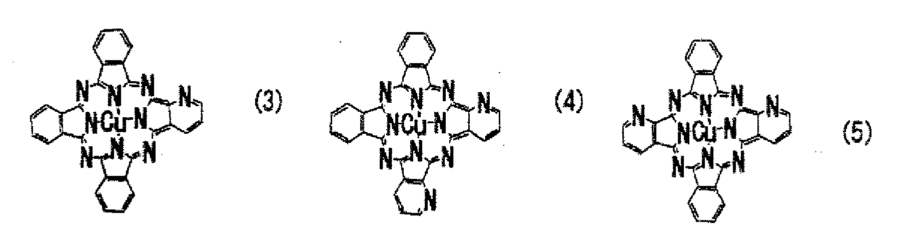

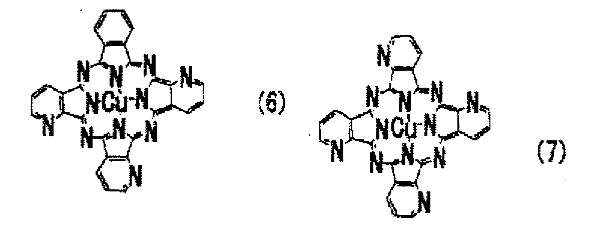

The tetraazaporphyrin compounds of the invention include a part or the whole of such a large number of isomers.

Specific examples of the tetraazaporphyrin compounds of the invention as represented by the general formula (1) given above are shown in Table 1. These specific examples are, however, by no means limitative of the scope of the compounds of the invention.

**Table 1**

| Specific example No. | Number of 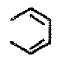 | Number of 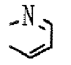 | n | R1 | R2 |
|---|---|---|---|---|---|
| 1 | 3 | 1 | 1 | H | -C₂H₄OC₂H₅ |
| 2 | 3 | 1 | 2 | H | -C₂H₄OC₂H₅ |
| 3 | 3 | 1 | 3 | H | -C₂H₄OC₂H₅ |
| 4 | 3 | 1 | 4 | H | -C₂H₄OC₂H₅ |
| 5 | 3 | 1 | 1 | -C₂H₄OC₂H₅ | -C₂H₄OC₂H₅ |
| 6 | 3 | 1 | 1 | H | -C₃H₆OC₄H₉ |
| 7 | 3 | 1 | 2 | H | -C₃H₆OC₄H₉ |
| 8 | 3 | 1 | 3 | H | -C₃H₆OC₄H₉ |
| 9 | 3 | 1 | 4 | H | -C₃H₆OC₄H₉ |
| 10 | 3 | 1 | 1 | -C₂H₄0CH₃ | -C₂H₄OCH₃ |
| 11 | 3 | 1 | 2 | -C₂H₄OCH₃ | -C₂H₄OCH₃ |
| 12 | 3 | 1 | 3 | -C₂H₄0CH₃ | -C₂H₄OCH₃ |
| 13 | 3 | 1 | 4 | -C₂H₄0CH₃ | -C₂H₄OCH₃ |
| 14 | 3 | 1 | 2 | -C₄H₉ | -C₄H₉ |
| 15 | 3 | 1 | 1 | H | 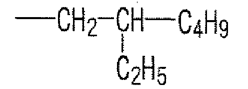 |
| 16 | 3 | 1 | 2 | H | 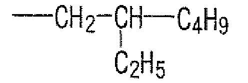 |
| 17 | 3 | 1 | 3 | H | 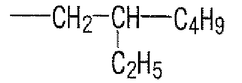 |
| 18 | 3 | 1 | 4 | H | 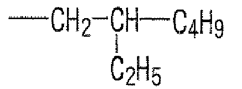 |
| 19 | 3 | 1 | 1 | H | 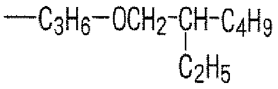 |
| 20 | 3 | 1 | 2 | H | 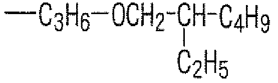 |
| 21 | 3 | 1 | 3 | H | 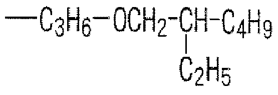 |
| 22 | 3 | 1 | 4 | H | 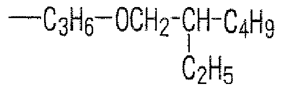 |
| 23 | 3 | 1 | 3 | H | -C₂H₄OC₂H₄OC₂H₅ |
| 24 | 3 | 1 | 4 | -C₂H₅ | -C₂H₄OC₂H₅ |
| 25 | 3 | 1 | 1 | -i-C₃H₇ | 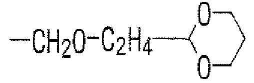 |
| 26 | 3 | 1 | 3 | H | 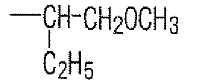 |
| 27 | 3 | 1 | 1 | H | 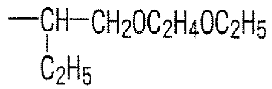 |
| 28 | 3 | 1 | 2 | H | 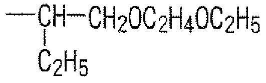 |
| 29 | 3 | 1 | 3 | H | 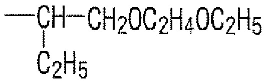 |
| 30 | 3 | 1 | 4 | H | 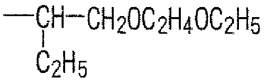 |
| 31 | 3 | 1 | 1 | H | 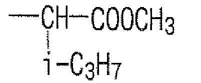 |
| 32 | 3 | 1 | 2 | H | 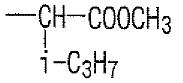 |
| 33 | 3 | 1 | 3 | H | 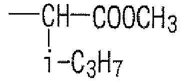 |
| 34 | 3 | 1 | 4 | H | 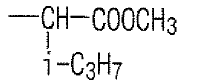 |
| 35 | 3 | 1 | 3 | -n-C₈H₁₇ | 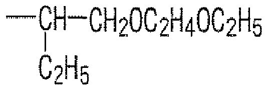 |
| 36 | 3 | 1 | 4 | H | 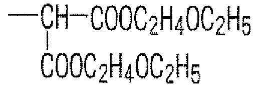 |
| 37 | 3 | 1 | 1 | -C₂H₄OC₂H₄OC₂H₅ | -C₂H₄OC₂H₄OC₂H₅ |
| 38 | 2 | 2 | 1 | H | -C₂H₄OC₂H₅ |
| 39 | 2 | 2 | 2 | H | -C₂H₄OC₂H₅ |
| 40 | 2 | 2 | 3 | H | -C₂H₄OC₂H₅ |
| 41 | 2 | 2 | 4 | H | -C₂H₄OC₂H₅ |
| 42 | 2 | 2 | 2 | -C₂H₄OC₂H₅ | -C₂H₄OC₂H₅ |
| 43 | 2 | 2 | 1 | H | -C₃H₆OC₄H₉ |
| 44 | 2 | 2 | 2 | H | -C₃H₆OC₄H₉ |
| 45 | 2 | 2 | 3 | H | -C₃H₆OC₄H₉ |
| 46 | 2 | 2 | 4 | H | -C₃H₆OC₄H₉ |
| 47 | 2 | 2 | 1 | -C₂H₄OCH₃ | -C₂H₄OCH₃ |
| 48 | 2 | 2 | 2 | -C₂H₄0CH₃ | -C₂H₄OCH₃ |
| 49 | 2 | 2 | 3 | -C₂H₄OCH₃ | -C₂H₄OCH₃ |
| 50 | 2 | 2 | 4 | -C₂H₄OCH₃ | -C₂H₄OCH₃ |
| 51 | 2 | 2 | 3 | -C₄H₉ | -C₄H₉ |
| 52 | 2 | 2 | 1 | H | 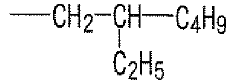 |
| 53 | 2 | 2 | 2 | H | 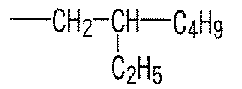 |
| 54 | 2 | 2 | 3 | H | 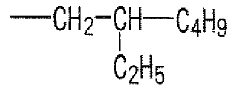 |
| 55 | 2 | 2 | 4 | H | 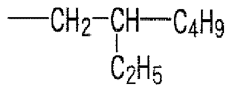 |
| 56 | 2 | 2 | 1 | H | 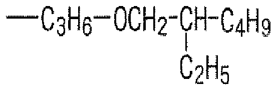 |
| 57 | 2 | 2 | 2 | H | 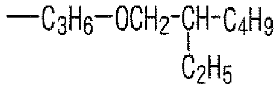 |
| 58 | 2 | 2 | 3 | H | 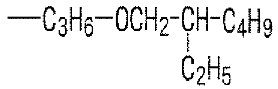 |
| 59 | 2 | 2 | 4 | H | 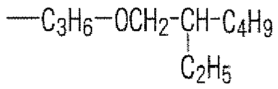 |
| 60 | 2 | 2 | 4 | H | -C₂H₄OC₂H₄OC₂H₅ |
| 61 | 2 | 2 | 1 | -C₂H₅ | -C₂H₄OC₂H₅ |
| 62 | 2 | 2 | 2 | -i-C₃H₇ | 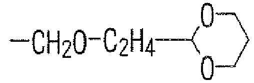 |
| 63 | 2 | 2 | 3 | H | 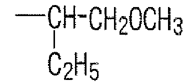 |
| 64 | 2 | 2 | 1 | H | 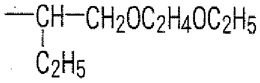 |
| 65 | 2 | 2 | 2 | H | 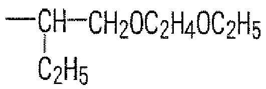 |
| 66 | 2 | 2 | 3 | H | 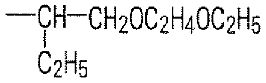 |
| 67 | 2 | 2 | 4 | H | 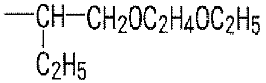 |
| 68 | 2 | 2 | 1 | H | 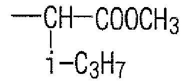 |
| 69 | 2 | 2 | 2 | H | 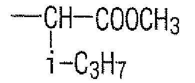 |
| 70 | 2 | 2 | 3 | H | 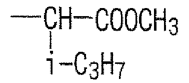 |
| 71 | 2 | 2 | 4 | H | 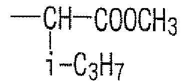 |
| 72 | 2 | 2 | 4 | -n-C₈H₁₇ | 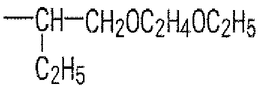 |
| 73 | 2 | 2 | 1 | H | 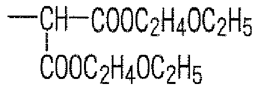 |
| 74 | 2 | 2 | 2 | -C₂H₄OC₂H₄OC₂H₅ | -C₂H₄OC₂H₄OC₂H₅ |
| 75 | 1 | 3 | 1 | H | -C₂H₄OC₂H₅ |
| 76 | 1 | 3 | 2 | H | -C₂H₄OC₂H₅ |
| 77 | 1 | 3 | 3 | H | -C₂H₄OC₂H₅ |
| 78 | 1 | 3 | 4 | H | -C₂H₄OC₂H₅ |
| 79 | 1 | 3 | 3 | -C₂H₄OC₂H₅ | -C₂H₄OC₂H₅ |
| 80 | 1 | 3 | 1 | H | -C₃H₆OC₄H₉ |
| 81 | 1 | 3 | 2 | H | -C₃H₆OC₄H₉ |
| 82 | 1 | 3 | 3 | H | -C₃H₆OC₄H₉ |
| 83 | 1 | 3 | 4 | H | -C₃H₆OC₄H₉ |
| 84 | 1 | 3 | 1 | -C₂H₄OCH₃ | -C₂H₄OCH₃ |
| 85 | 1 | 3 | 2 | -C₂H₄0CH₃ | -C₂H₄OCH₃ |
| 86 | 1 | 3 | 3 | -C₂H₄OCH₃ | -C₂H₄OCH₃ |
| 87 | 1 | 3 | 4 | -C₂H₄OCH₃ | -C₂H₄OCH₃ |
| 88 | 1 | 3 | 4 | -C₄H₉ | -C₄H₉ |
| 89 | 1 | 3 | 1 | H | 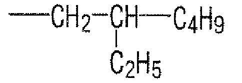 |
| 90 | 1 | 3 | 2 | H | 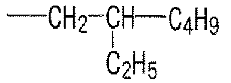 |
| 91 | 1 | 3 | 3 | H | 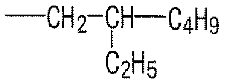 |
| 92 | 1 | 3 | 4 | H | 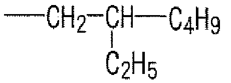 |
| 93 | 1 | 3 | 1 | H | 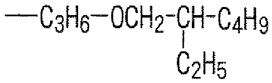 |
| 94 | 1 | 3 | 2 | H | 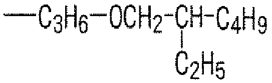 |
| 95 | 1 | 3 | 3 | H | 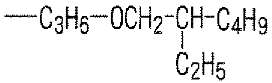 |
| 96 | 1 | 3 | 4 | H | 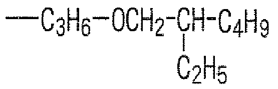 |
| 97 | 1 | 3 | 1 | H | -C₂H₄OC₂H₄OC₂H₅ |
| 98 | 1 | 3 | 2 | -C₂H₅ | -C₂H₄OC₂H₅ |
| 99 | 1 | 3 | 3 | -i-C₃H₇ | 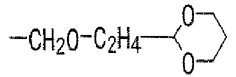 |
| 100 | 1 | 3 | 4 | H | 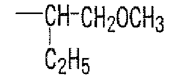 |
| 101 | 1 | 3 | 1 | H | 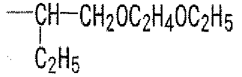 |
| 102 | 1 | 3 | 2 | H | 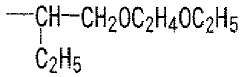 |
| 103 | 1 | 3 | 3 | H | 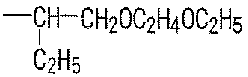 |
| 104 | 1 | 3 | 4 | H | 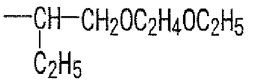 |
| 105 | 1 | 3 | 1 | H | -CH-C₂H₄COCH₃ |
| 106 | 1 | 3 | 2 | H | -CH-C₂H₄COCH₃ |
| 107 | 1 | 3 | 3 | H | -CH-C₂H₄COC₃H₇ |
| 108 | 1 | 3 | 4 | H | -CH-C₂H₄COC₃H₇ |
| 109 | 1 | 3 | 1 | -n-C₈H₁₇ | 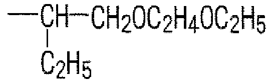 |
| 110 | 1 | 3 | 2 | H | 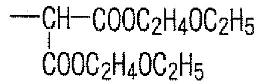 |
| 111 | 1 | 3 | 3 | -C₂H₄0C₂H₄0C₂H₅ | -C₂H₄0C₂H₄0C₂H₅ |
| 112 | 0 | 4 | 1 | H | -C₂H₄OC₂H₅ |
| 113 | 0 | 4 | 2 | H | -C₂H₄OC₂H₅ |
| 114 | 0 | 4 | 3 | H | -C₂H₄OC₂H₅ |
| 115 | 0 | 4 | 4 | H | -C₂H₄0C₂H₅ |
| 116 | 0 | 4 | 4 | -C₂H₄OC₂H₅ | -C₂H₄OC₂H₅ |
| 117 | 0 | 4 | 1 | H | -C₃H₆OC₄H₉ |
| 118 | 0 | 4 | 2 | H | -C₃H₆OC₄H₉ |
| 119 | 0 | 4 | 3 | H | -C₃H₆OC₄H₉ |
| 120 | 0 | 4 | 4 | H | -C₃H₆OC₄H₉ |
| 121 | 0 | 4 | 1 | -C₂H₄OCH₃ | -C₂H₄OCH₃ |
| 122 | 0 | 4 | 2 | -C₂H₄OCH₃ | -C₂H₄OCH₃ |
| 123 | 0 | 4 | 3 | -C₂H₄OCH₃ | -C₂H₄OCH₃ |
| 124 | 0 | 4 | 4 | -C₂H₄OCH₃ | -C₂H₄OCH₃ |
| 125 | 0 | 4 | 1 | -C₄H₉ | -C₄H₉ |
| 126 | 0 | 4 | 1 | H | 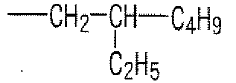 |
| 127 | 0 | 4 | 2 | H | 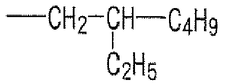 |
| 128 | 0 | 4 | 3 | H | 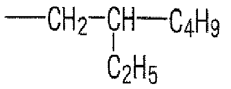 |
| 129 | 0 | 4 | 4 | H | 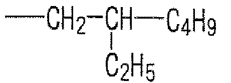 |
| 130 | 0 | 4 | 1 | H | -CHCOCH₃ |
| 131 | 0 | 4 | 2 | H | -CHCOCH₃ |
| 132 | 0 | 4 | 3 | H | -CHCOOC₃H₇ |
| 133 | 0 | 4 | 4 | H | -CHCOOC₃H₇ |
| 134 | 0 | 4 | 2 | H | -C₂H₄OC₂H₄OC₂H₅ |
| 135 | 0 | 4 | 3 | -C₂H₅ | -C₂H₄OC₂H₅ |
| 136 | 0 | 4 | 4 | -i-C₃H₇ | 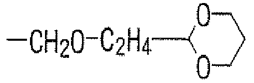 |
| 137 | 0 | 4 | 1 | H | 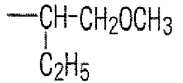 |
| 138 | 0 | 4 | 1 | H | 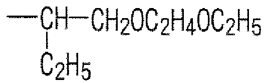 |
| 139 | 0 | 4 | 2 | H | 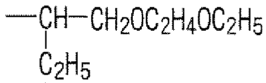 |
| 140 | 0 | 4 | 3 | H | 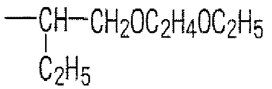 |
| 141 | 0 | 4 | 4 | H | 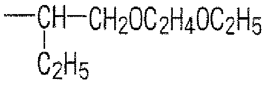 |
| 142 | 0 | 4 | 1 | H | 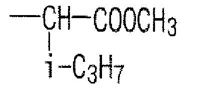 |
| 143 | 0 | 4 | 2 | H | 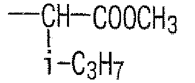 |
| 144 | 0 | 4 | 3 | H | 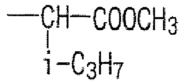 |
| 145 | 0 | 4 | 4 | H | 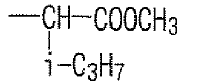 |
| 146 | 0 | 4 | 2 | -n-C₈H₁₇ | 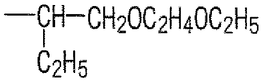 |
| 147 | 0 | 4 | 3 | H | 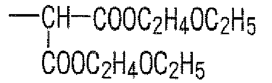 |
| 148 | 0 | 4 | 4 | -C₂H₄OC₂H₄OC₂H₅ | -C₂H₄OC₂H₄OC₂H₅ |
| 1 4 9 | 3 | 1 | 4 | -C₁₀H₂₁ | H |
| 150 | 3 | 1 | 3 | -C₁₂H₂₅ | -C₁₂H₂₅ |
| 151 | 3 | 1 | 4 | -C₁₂H₂₅ | -C₂H₄OC₂H₅ |
| 152 | 3 | 1 | 1 | 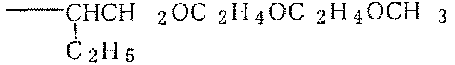 | H |
| 153 | 3 | 1 | 3 | 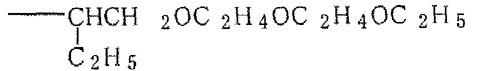 | -C₂H₅ |
| 154 | 3 | 1 | 1 | 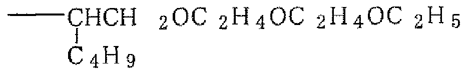 | H |
| 155 | 3 | 1 | 1 | 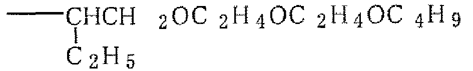 | H |
| 156 | 3 | 1 | 2 | 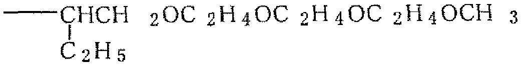 | H |
| 157 | 3 | 1 | 3 | 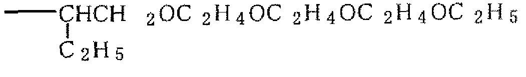 | H |

### (Method of producing the tetraazaporphyrin compounds)

A method of producing the tetraazaporphyrin compounds of the invention as represented by the above general formula (1) is described below.

A typical production method is summarized as follows.
(1) The basic skeleton of a tetraazaporphyrin compound is produced by mixing phthalic acid (which may optionally has a substituent(s) other than -SO₂NR¹R²) with pyridine-2,3-dicarboxylic acid (which may optionally have a substituent(s) other than -SO₂NR¹R²) and allowing the reaction to proceed.
(2) The compound obtained is then chlorosulfonylated.
(3) The chlorosulfonylated tetraazaporphyrin is amidated by reacting with an amine to give the desired compound.

This production method is now described in further detail.
(1) Tetraazaporphyrin ring formation step
   As for the tetraazaporphyrin ring formation method, phthalic acid and pyridine-2,3-dicarboxylic acid and a copper powder, copper oxide or copper salt are heated at 120-300°C without using any solvent or in such as solvent as tetraline, 1-chloronaphthalene, nitrobenzene, trichlorobenzene or DMI in the presence of gaseous ammonia or urea and in the presence of ammonium molybdate or the like as a catalyst, whereby the desired ring is obtained.
   The mixing ratio between phthalic acid and pyridine-2,3-dicarboxylic acid in the above ring formation reaction is 3.99: 0.01 to 0:4, preferably 3:1 to 0:4. The dicyano form or acid anhydride of phthalic acid or gyridine.-2,3-dicarboxylic acid can also be used in lieu of phthalic acid or pyridine-2, 3-dicarboxylic acid, respectively.
(2) Chlorosulfonylation step
   The tetraazaporphyrin compound obtained in the above step (1) is added portionwise to an amount of 5-20 times the weight thereof of chlorosulfonic acid while maintaining the temperature at 20°C or below. The resulting mixture is stirred at the same temperature for 1 hour and then the reaction is allowed to proceed at 130-135°C for 4 hours. The reaction mixture is cooled to 80°C, and an amount of 2-5 times the weight of the tetraazaporphyrin compound of thionyl chloride is added dropwise over 1-2 hours while maintaining the temperature at 70-80°C. The resulting mixture is stirred at the same temperature for 2-10 hours and then cooled to 15-20°C, and stirred at that temperature for 12 hours. This reaction mixture is discharged portionwise into an amount of 50-200 times the weight of the chlorosulfonic acid used of ice water, the resulting precipitate is filtered off and washed with ice water until the washings become neutral to give the sulfonyl chloride form of the tetraazaporphyrin compound.
   The above-mentioned reaction conditions are the reaction conditions for mainly obtaining tetrasulfonyl chlorides. When mono-, di- or tri-substituted sulfonyl chlorides are desired, this becomes possible by employing milder reaction conditions in chlorosulfonic acid. Namely, this can be accomplished by lowering the reaction temperature or shorten the reaction time.
(3) Sulfonamidation step
   The tetraazaporphyrinsulfonyl chloride obtained as described above is suspended in ice water and an organic amine represented by the formula (8):

   NHR¹R² (8)

   (wherein R¹ and R² are as defined above referring to the formula (1)) (in an amount 2-8 moles per mole of the tetraazaporphyrin compound) is added while maintaining the temperature at 15°C or below. After completion of the dropping, the mixture is stirred at 20-30°C for 15-24 hours, and the product is filtered off, washed with water and dried to give the desired tetraazaporphyrinsulfonamide compound of formula (1).
   In many cases, the thus-obtained product occurs as a mixture of a plurality of isomers, as mentioned above. Even such a mixture can accomplish the object of the invention and thus falls within the scope of the present invention.
   If necessary, this mixture can be purified in the conventional manner, for example recrystallization from such an organic solvent as ethyl acetate, acetone or methanol and/or further purification by column chromatography, to give an individual single product.

### EXAMPLES

The following examples illustrate the present invention more specifically. These examples are, however, by no means limitative of the scope of the invention.

### [Example 1] Synthesis of Specific Example 44 tetraazaporphyrin compound

Phthalic anhydride (7.4 g), 8.4 g of pyridine-2,3-dicarboxylic acid, 36 g of urea, 2.5 g of cuprous chloride and 0.4 g of ammonium molybdate were suspended in 70 mL of 1-chloronaphthalene, and the mixture was stirred at 190°C-220°C for 5 hours. The reaction mixture was poured into 250 mL of methanol, and the precipitate was filtered off, washed in sequence with methanol, water and acetone and then dried to give 12.8 g of a blue unsubstituted tetraazaporphyrin compound.

A 12-g portion of this unsubstituted tetraazaporphyrin compound was added portionwise over 30 minutes to 120 g of chlorosulfonic acid at 20°C or below. Then, the temperature was raised to 70-80°C, the mixture was stirred at that temperature for 1 hour, the temperature was then raised to 130-135°C over 2 hours, the reaction was allowed to proceed at that temperature for 4 hours, and the reaction mixture was cooled to 80°C. Further, 20 g of thionyl chloride was added dropwise over 1 hour while maintaining the temperature at 70-80°C, and the resulting mixture was stirred at 70-80°C for 2 hours. The reaction mixture was cooled to 15-20°C and stirred at that temperature for 12 hours. The reaction mixture was discharged portionwise into 1000 g of ice water, and the precipitate was collected by filtration, washed with ice water until the washings became neutral, to give the sulfonyl chloride of the tetraazaporphyrin compound in the form of a hydrous paste. This was immediately poured into 400 g of ice water, the mixture was stirred at 10°C or below for 30 minutes for dispersion, 20 g of 3-butoxypropylamine was added dropwise. Then, the temperature was raised to 20-30°C and, after 18 hours of stirring at that temperature, the product was collected by filtration and dispersed in 200 g of water, followed by filtration. This dispersion/filtration procedure was carried out twice. After 16 hours of drying at 60°C, 10 g of a blue powder was obtained. The powder was further subjected to recrystallization from ethyl acetate to give 6.4 of blue crystals.

Upon fluorescent X ray analysis, it was confirmed, based on the intensity ratio between the tetraazaporphyrin skeleton center atom copper and the sulfonamide sulfur atom, that these blue crystals contained, on an average, 1.9 sulfonamide groups per molecule.

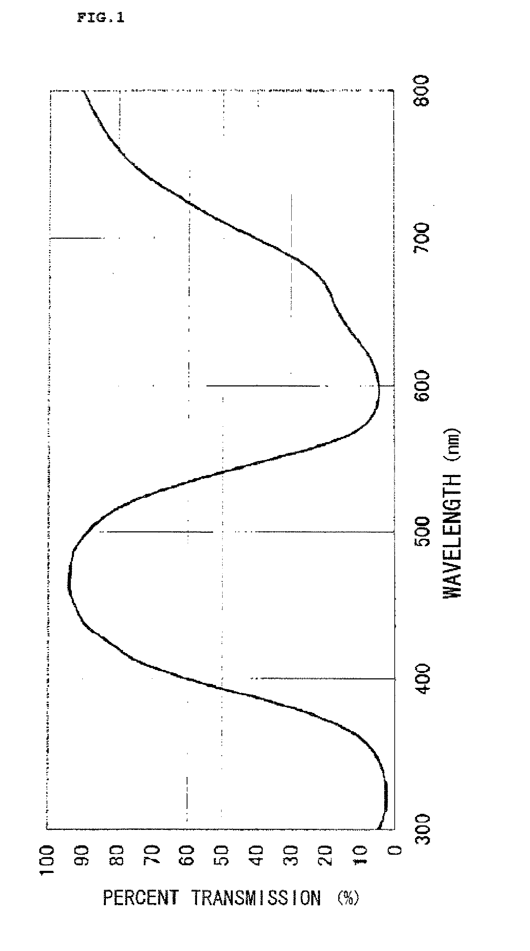

Using 0.3 g of these blue crystals, 0.5 g of an acrylic resin (Delpet 80N; product of Asahi Chemical Industry) and 9.5 g of ethyl acetate, a pigment resin solution was prepared and applied to a glass sheet by the spin coat method. After 1 hour of drying at 60°C, the coat was subjected to transmission spectrum measurement. The transmission spectrum obtained is shown in Fig. 1.

The blue crystals (5 g) were subjected to silica gel column chromatography for separation/purification using a toluene-methanol mixed solvent to give 1.5 g of a purified blue powder. Upon fluorescent X ray analysis, it was confirmed, based on the intensity ratio between the tetraazaporphyrin skeleton center atom copper and the sulfonamide sulfur atom, that this purified blue powder contained 2 sulfonamide groups per molecule. Based on the result of FD-MS analysis, namely m/z = 964, 771, it was confirmed that the main component of this product was a compound identified as Specific Example 44 resulting from introduction of 2 pyridine rings.

### [Example 2] Synthesis of Specific Example 8 tetraazaporphyrin compound

Phthalic anhydride (11.1 g), 4.2 g of pyridine-2,3-dicarboxylic acid, 36 g of urea, 2.5 g of cuprous chloride and 0.4 g of ammonium molybdate were suspended in 70 mL of 1,3-dimethyl-2-imidazolidinone, and the mixture was stirred at 190°C-220°C for 5 hours. The reaction mixture was subjected to after-treatment in the same manner as in Example 1 to give 12.3 g of an unsubstituted tetraazaporphyrin compound. This was subjected to chlorosulfonation, amidation and after-treatment in the same manner as in Example 1 to give 6.1 g of blue crystals.

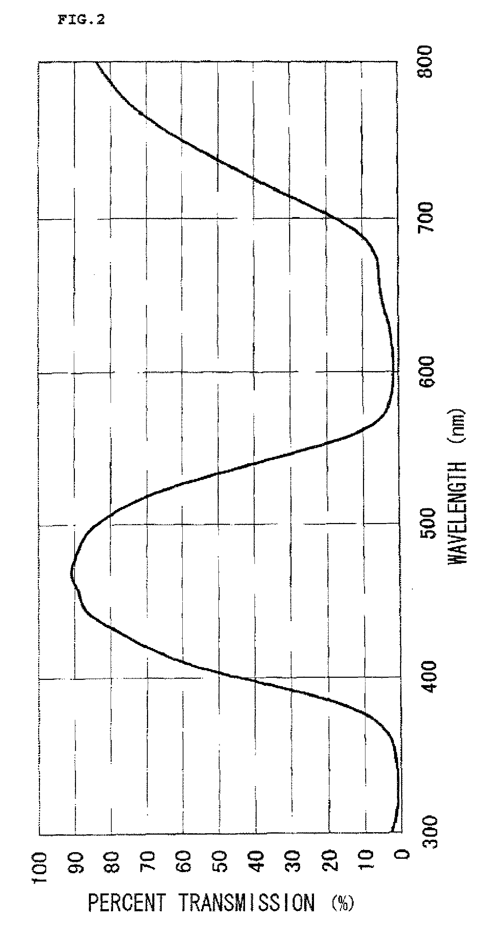

Upon fluorescent X ray analysis, it was confirmed, based on the intensity ratio between the tetraazaporphyrin skeleton center atom copper and the sulfonamide sulfur atom, that these blue crystals contained, on an average, 2.9 sulfonamide groups per molecule. Transmission spectrum measurement of the blue crystals was carried out in the same manner as in Example 1. The transmission spectrum obtained is shown in Fig. 2.

The blue crystals (5 g) were subjected to silica gel column chromatography for separation/purification using a toluene-methanol mixed solvent to give 1.7 g of a purified blue powder. Upon fluorescent X ray analysis, it was confirmed, based on the intensity ratio between the tetraazaporphyrin skeleton center atom copper and the sulfonamide sulfur atom, that this purified blue powder contained 3 sulfonamide groups per molecule. Based on the result of FD-MS analysis, namely m/z = 1156, 963, it was confirmed that the main component of this product was a compound identified as Specific Example 8 resulting from introduction of 1 pyridine ring.

### [Example 3] Synthesis of Specific Example 93 tetraazaporphyrin compound

Phthalic anhydride (3.7 g), 12.5 g of pyridine-2,3-dicarboxylic acid, 36 g of urea, 2.5 g of cuprous chloride and 0.4 g of ammonium molybdate were suspended in 70 mL of 1,3-dimethyl-2-imidazolidinone, and the mixture was stirred at 190°C-220°C for 5 hours. The reaction mixture was subjected to after-treatment in the same manner as in Example 1 to give 11.5 g of an unsubstituted tetraazaporphyrin compound. This was subjected to chlorosulfonation, amidation and after-treatment in the same manner as in Example 1 except that 25 g of 3-(2-ethylhexyloxy)propylamine was used in lieu of 20 g of 3-butoxypropylamine, to give 5.7 g of blue crystals. Upon fluorescent X ray analysis, it was confirmed, based on the intensity ratio between the tetraazaporphyrin skeleton center atom copper and the sulfonamide sulfur atom, that these blue crystals contained, on an average, 1.3 sulfonamide groups per molecule.

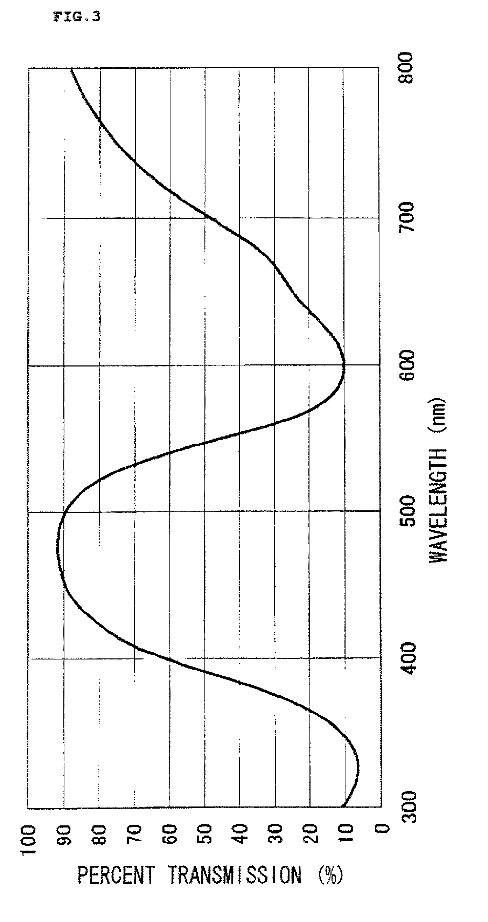

Transmission spectrum measurement of the blue crystals was carried out in the same manner as in Example 1. The transmission spectrum obtained is shown in Fig. 3. These blue crystals (4 g) were subjected to silica gel column chromatography for separation/purification using a toluene-methanol mixed solvent to give 2.2 g of a purified blue powder. Upon fluorescent X ray analysis, it was confirmed, based on the intensity ratio between the tetraazaporphyrin skeleton center atom copper and the sulfonamide sulfur atom, that this purified blue powder contained 1 sulfonamide group per molecule. Based on the result of FD-MS analysis, namely m/z = 828, 1076, it was confirmed that the main component of this product was a compound identified as Specific Example 93 resulting from introduction of 3 pyridine rings.

### [Example 4] Synthesis of Specific Example 48 tetraazaporphyrin compound

Blue crystals (5.7 g) were obtained in the same manner as in Example 1 except that 25 g of bis(2-methoxyethyl)amine was used in lieu of 20 g of 3-butoxypropylamine.

Upon fluorescent X ray analysis, it was confirmed, based on the intensity ratio between the tetraazaporphyrin skeleton center atom copper and the sulfonamide sulfur atom, that these blue crystals contained, on an average, 2.1 sulfonamide groups per molecule.

The blue crystals (5 g) were subjected to silica gel column chromatography for separation/purification using a toluene-methanol mixed solvent to give 2.6 g of a purified blue powder. Upon fluorescent X ray analysis, it was confirmed, based on the intensity ratio between the tetraazaporphyrin skeleton center atom copper and the sulfonamide sulfur atom, that this purified blue powder contained 2 sulfonamide groups per molecule. Based on the result of FD-MS analysis, namely m/z = 968, 773, it was confirmed that the main component of this product was a compound identified as Specific Example 48 resulting from introduction of 2 pyridine rings.

### [Example 5] Synthesis of Specific Example 17 tetraazaporphyrin compound

Blue crystals (6.1 g) were obtained in the same manner as in Example 2 except that 20 g of 2-ethylhexylamine was used in lieu of 20 g of 3-butoxypropylamine. Upon fluorescent X ray analysis, it was confirmed, based on the intensity ratio between the tetraazaporphyrin skeleton center atom copper and the sulfonamide sulfur atom, that these blue crystals contained, on an average, 2.8 sulfonamide groups per molecule.

The blue crystals (5 g) were subjected to silica gel column chromatography for separation/purification using a toluene-methanol mixed solvent to give 1.9 g of a purified blue powder. Upon fluorescent X ray analysis, it was confirmed, based on the intensity ratio between the tetraazaporphyrin skeleton center atom copper and the sulfonamide sulfur atom, that this purified blue powder contained 3 sulfonamide groups per molecule. Based on the result of FD-MS analysis, namely m/z = 1150.959, it was confirmed that the main component of this product was a compound identified as Specific Example 17 resulting from introduction of 1 pyridine ring.

### [Example 6] Synthesis of Specific Example 26 tetraazaporphyrin compound

Blue crystals (3.7 g) were obtained in the same manner as in Example 2 except that 20 g of 2-amino-1-methoxybutane was used in lieu of 20 g of 3-butoxypropylamine. Upon fluorescent X ray analysis, it was confirmed, based on the intensity ratio between the tetraazaporphyrin skeleton center atom copper and the sulfonamide sulfur atom, that these blue crystals contained, on an average, 2.9 sulfonamide groups per molecule.

The blue crystals (3 g) were subjected to silica gel column chromatography for separation/purification using a toluene-methanol mixed solvent to give 1.2 g of a purified blue powder. Upon fluorescent X ray analysis, it was confirmed, based on the intensity ratio between the tetraazaporphyrin skeleton center atom copper and the sulfonamide sulfur atom, that this purified blue powder contained 3 sulfonamide groups per molecule. Based on the result of FD-MS analysis, namely m/z = 1072, 907, it was confirmed that the main component of this product was a compound identified as Specific Example 26 resulting from introduction of 1 pyridine ring.

### [Example 7] Synthesis of Specific Example 29 tetraazaporphyrin compound

Blue crystals (5.5 g) were obtained in the same manner as in Example 2 except that 5 g of 2-amino-1-(2-ethoxyethoxy) butane and 10 g of triethylamine were used in lieu of 20 g of 3-butoxypropylamine. Upon fluorescent X ray analysis, it was confirmed, based on the intensity ratio between the tetraazaporphyrin skeleton center atom copper and the sulfonamide sulfur atom, that these blue crystals contained, on an average, 3.1 sulfonamide groups per molecule.

The blue crystals (4 g) were subjected to silica gel column chromatography for separation/purification using a toluene-methanol mixed solvent to give 1.7 g of a purified blue powder. Upon fluorescent X ray analysis, it was confirmed, based on the intensity ratio between the tetraazaporphyrin skeleton center atom copper and the sulfonamide sulfur atom, that this purified blue powder contained 3 sulfonamide groups per molecule. Based on the result of FD-MS analysis, namely m/z = 1246, 1023, it was confirmed that the main component of this product was a compound identified as Specific Example 29 resulting from introduction of 1 pyridine ring.

### [Example 8] Synthesis of Specific Example 33 tetraaxaporphyrin compound

Blue crystals (6.4 g) were obtained in the same manner as in Example 2 except that 20 g of L-valine methyl ester hydrochloride and 15 g of triethylamine were used in lieu of 20 g of 3-butoxypropylamine. Upon fluorescent X ray analysis, it was confirmed, based on the intensity ratio between the tetraazaporphyrin skeleton center atom copper and the sulfonamide sulfur atom, that these blue crystals contained, on an average, 3.1. sulfonamide groups per molecule.

The blue crystals (5 g) were subjected to silica gel column chromatography for separation/purification using a toluene-methanol mixed solvent to give 2.2 g of a purified blue powder. Upon fluorescent X ray analysis, it was confirmed, based on the intensity ratio between the tetraazaporphyrin skeleton center atom copper and the sulfonamide sulfur atom, that this purified blue powder contained 3 sulfonamide groups per molecule. Based on the result of FD-MS analysis, namely m/z = 1156, 963, it was confirmed that the main component of this product was a compound identified as Specific Example 33 resulting from introduction of 1 pyridine ring.

### [Comparative Example 1] Copper phthalocyanine (12 g; product of Tokyo Kasei) was added portionwise over 30 minutes to 120 g of chlorosulfonic acid at 20°C or below. Then, the temperature was raised to 70-80°C and, after 1 hour of stirring at that temperature, the temperature was raised to 130-150°C over 2 hours, and the reaction was allowed to proceed at that temperature for 4 hours and, then, the mixture was cooled to 80°C. Further, 20 g of thionyl chloride was added dropwise over 1 hour while maintaining the temperature at 70-80°C, and the resulting mixture was stirred at 70-80°C for 4 hours and then cooled to 15-20°C and stirred at that temperature for 12 hours.

The reaction mixture was discharged portionwise into 1000 g of ice water, and the precipitate was collected by filtration and washed with ice water until the washings became neutral, to give phthaloayaninesulfonyl, chloride in the form of a hydrous paste. This was immediately poured into 400 g of ice water and, after 30 minutes of stirring at 10°C or below, 20 g of 3-butoxypropylamine was added dropwise. Then, the temperature was raised to 20-30°C and, after 18 hours of stirring at that temperature, the product was collected by filtration and subjected to two repetitions of a procedure comprising dispersion in 200 g of water and recovery by filtration and then dried at 60°C for 15 hours to give 15 g of a blue powder, which was recrystallized from ethyl acetate to give 6.4 g of blue crystals. Upon fluorescent X ray analysis, the average number of sulfonamide groups was found to be 3.8 per molecule based on the intensity ratio between the phthalocyanine skeleton center atom copper and the sulfonamide group sulfur atom.

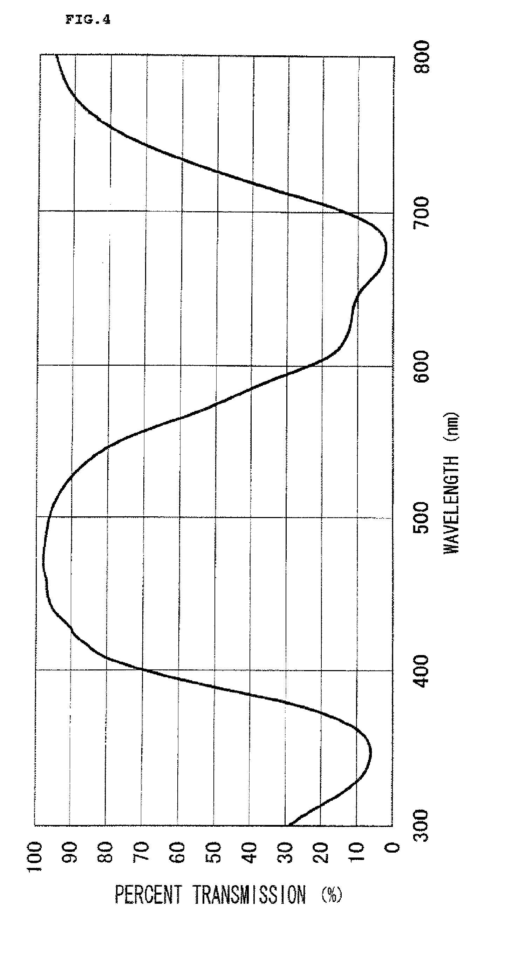

Using the blue crystals, transmission spectrum measurement was carried out in the same manner as in Example 1. The transmission spectrum obtained is shown in Fig. 4.

While the transmission spectra of the tetraazaporphyrin compounds of the invention show strong absorption at about 600 nm and the compounds have a brilliant blue color, the compound of this comparative example showed an absorption peak at about 680 nm and had a greenish blue color.

### INDUSTRIAL APPLICABILITY

The tetraazaporphyrin compounds of the invention have a brilliant blue color and show strong absorption at about 600 nm and are highly soluble in organic solvents and resins and, therefore, they are suited for use in highly transparent toners, in brilliant blue inks for ink-jet printers and in coloring polymer materials.

## Claims

1. A tetraazaporphyrin compound represented by the following general formula (1): 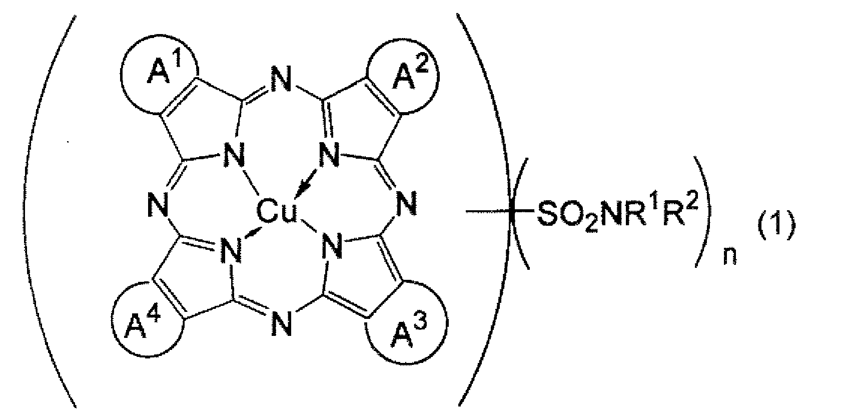 (In the formula (1), the rings represented by A¹, A², A³ and A⁴ each independently represents 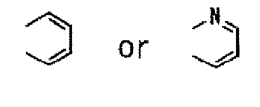 and at least one of A¹, A², A³ and A⁴ is 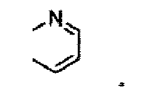 R¹ and R² each independently represents a hydrogen atom or a substituted or unsubstituted alkyl group, and n represents an integer of 1-4, provided that when one of R¹ and R² is a hydrogen atom, the other is other than a hydrogen atom. The rings A¹, A², A³ and A⁴ each independently may have a substituent(s) other than -SO₂NR¹R²-)

2. A tetraazaporphyrin, compound according to Claim 1, wherein R¹ and R² each independently is a hydrogen atom (provided that one of R¹ and R² is a hydrogen atom, the other is other than a hydrogen atom); an unsubstituted alkyl group; or a substituted alkyl group containing one or more oxygen atoms in the form of one or more ether bonds, carbonyl bonds and/or ester bonds.

3. A tetraazaporphyrin compound according to Claim 1 or 2,
wherein R¹ and R² each independently is a hydrogen atom (provided that one of R¹ and R² is a hydrogen atom, the other is other than a hydrogen atom); an unsubstituted alkyl group containing 1-12 carbon atoms; or a substituted alkyl group containing 2-12 carbon atoms and containing 1-4 oxygen atoms in the form of one or more ether bonds, carbonyl bonds and/or ester bonds.

4. A tetraazaporphyrin compound according to any of Claims 1-3, wherein at least one of R¹ and R² is a substituted alkyl group containing 2-12 carbon atoms and containing 1-4 oxygen atoms in the form of one or more ether bonds, carbonyl bonds and/or ester bonds.

5. A tetraazaporphyrin compound according to Claim 4, wherein at least one of R¹ and R² is a substituted alkyl group represented by the following general formula (2): 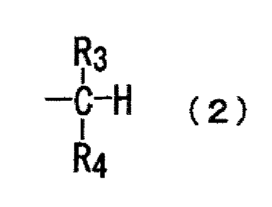 (In the formula (2), R³ and R⁴ each independently is a hydrogen atom; an unsubstituted alkyl group; an alkyl group containing one or more oxygen atoms in the form of one or more ether bonds, carbonyl bonds and/or ester bonds; an alkylcarbonyl group; or an alkoxycarboyl group, provided that at least one of R³ and R⁴ is an alkyl group containing one or more oxygen atoms in the form of one or more ether bonds, carbonyl bonds and/or ester bonds; an alkylcarbonyl group; or an alkoxycarboyl group.)

6. A tetraazaporphyrin compound according to Claim 5, wherein R³ and R⁴ each independently is a hydrogen atom; an unsubstituted alkyl group containing 1-8 carbon atoms; a substituted alkyl group containing 2-10 carbon atoms and containing 1-4 oxygen atoms in the form of one or more ether bonds, carbonyl bonds and/or ester bonds; an alkylcarbonyl group containing 2-10 carbon atoms; or an alkoxycarboyl group containing 2-10 carbon atoms, provided that at least one of R³ and R⁴ is an alkyl group containing one or more oxygen atoms in the form of one or more ether bonds, carbonyl bonds and/or ester bonds; an alkylcarbonyl group; or an alkoxycarboyl group.)

## Amended claims

### Amended claims under Art. 19.1 PCT

**1.** (Canceled)

**2.** (Amended) A tetraazaporphyrin compound represented by the following general formula (1): 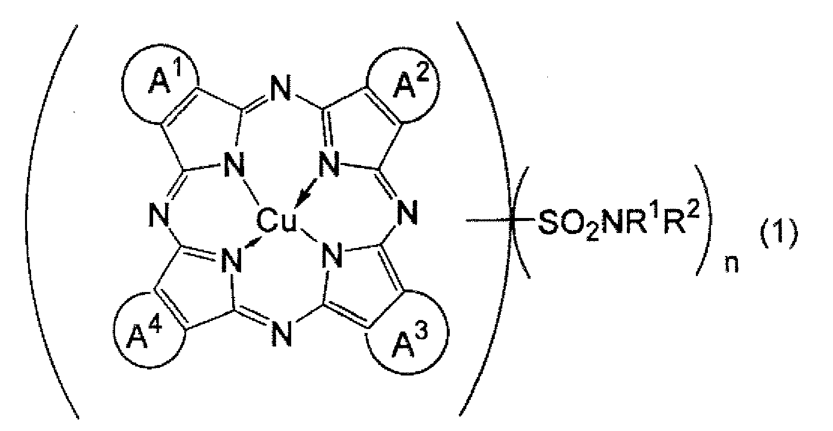 (In the formula (1), the rings represented by A¹, A², A³ and A⁴ each independently represents 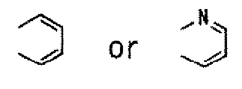 and at least one of A¹, A², A³ and A⁴ is 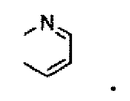 R¹ and R² each independently represents a hydrogen atom or a substituted or unsubstituted alkyl group, and n represents an integer of 1-4, provided that when one of R¹ and R² is a hydrogen atom, the other is other than a hydrogen atom. The rings A¹, A², A³ and A⁴ each independently may have a substituent (s) other than -SO₂NR¹R².) wherein R¹ and R² each independently is a hydrogen atom (provided that one of R¹ and R² is a hydrogen atom, the other is other than a hydrogen atom); an unsubstituted alkyl group; or a substituted alkyl group containing one or more oxygen atoms in the form of one or more ether bonds, carbonyl bonds and/or ester bonds.

**3.** A tetraazaporphyrin compound according to Claim 1 or 2, wherein R¹ and R² each independently is a hydrogen atom (provided that one of R¹ and R² is a hydrogen atom, the other is other than a hydrogen atom); an unsubstituted alkyl group containing 1-12 carbon atoms; or a substituted alkyl group containing 2-12 carbon atoms and containing 1-4 oxygen atoms in the form of one or more ether bonds, carbonyl bonds and/or ester bonds.

**4.** A tetraazaporphyrin compound according to any of Claims 1-3, wherein at least one of R¹ and R² is a substituted alkyl group containing 2-12 carbon atoms and containing 1-4 oxygen atoms in the form of one or more ether bonds, carbonyl bonds and/or ester bonds.

**5.** A tetraazaporphyrin compound according to Claim 4, wherein at least one of R¹ and R² is a substituted alkyl group represented by the following general formula (2): 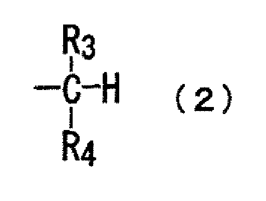 (In the formula (2), R³ and R⁴ each independently is a hydrogen atom; an unsubstituted alkyl group; an alkyl group containing one or more oxygen atoms in the form of one or more ether bonds, carbonyl bonds and/or ester bonds; an alkylcarbonyl group; or an alkoxycarboyl group, provided that at least one of R³ and R⁴ is an alkyl group containing one or more oxygen atoms in the form of one or more ether bonds, carbonyl bonds and/or ester bonds; an alkylcarbonyl group; or an alkoxycarboyl group.)

**6.** A tetraazaporphyrin compound according to Claim 5, wherein R³ and R⁴ each independently is a hydrogen atom; an unsubstituted alkyl group containing 1-8 carbon atoms; a substituted alkyl group containing 2-10 carbon atoms and containing 1-4 oxygen atoms in the form of one or more ether bonds, carbonyl bonds and/or ester bonds; an alkylcarbonyl group containing 2-10 carbon atoms; or an alkoxyoarboyl group containing 2-10 carbon atoms, provided that at least one of R³ and R⁴ is an alkyl group containing one or more oxygen atoms in the form of one or more ether bonds, carbonyl bonds and/or ester bonds; an alkylcarbonyl group; or an alkoxycarboyl group.)
